Europäisches Patentamt

European Patent Office

Office européen des brevets

(19)

(11) Veröffentlichungsnummer: **0 164 306**
**B1**

(12) **EUROPÄISCHE PATENTSCHRIFT**

(45) Veröffentlichungstag der Patentschrift: **25.07.90**

(21) Anmeldenummer: **85810173.6**

(22) Anmeldetag: **19.04.85**

(51) Int. Cl.⁵: **C 12 M 1/26,** B 01 F 13/08

(54) **Vorrichtung zur Aufbewahrung, zum Transport, zum Transfer und zur Probeentnahme von sterilen Lösungen.**

(30) Priorität: **08.06.84 CH 2800/84**

(43) Veröffentlichungstag der Anmeldung:
**11.12.85 Patentblatt 85/50**

(45) Bekanntmachung des Hinweises auf die
Patenterteilung:
**25.07.90 Patentblatt 90/30**

(84) Benannte Vertragsstaaten:
**AT BE CH DE FR GB IT LI LU NL SE**

(56) Entgegenhaltungen:
**DE-A-2 017 472**
**GB-A-1 238 401**
**US-A-2 837 320**

(73) Patentinhaber: **STAWAG**
**Wagistrasse 13**
**CH-8952 Schlieren (CH)**

(72) Erfinder: **Stapfer, Werner**
**Weidstrasse 9**
**CH-8103 Unterengstringen (CH)**

(74) Vertreter: **Herrmann, Peter Johannes et al**
**c/o PPS Polyvalent Patent Service AG**
**Mellingerstrasse 1**
**CH-5400 Baden (CH)**

Courier Press, Leamington Spa, England.

EP 0 164 306 B1

**Beschreibung**

Die Erfindung betrifft eine Vorrichtung zur Aufbewahrung, zum Transport, zum Transfer und zur Probeentnahme von sterilen Lösungen und/oder Suspensionen, insbesondere als Inokuliergefäss mit einem sterilisierbaren Edelstahlbehälter, dessen hermetisch abschliessbarer Deckel mehrere Bohrungen aufweist, wovon eine zentral gelegen ist und eine gasdicht eingelassene hohle Rührachse besitzt, an deren unterem Ende ein Magnetrührkörper drehbeweglich gelagert ist.

Vorrichtungen zur Aufbewahrung und zum Transport von sterilen Lösungen sind aus der Mikrobiologie bekannt. Dafür dienen in der Regel Behälter aus Glas oder Edelstahl, die mit Dichtungen versehen sind und nach der Sterilisation bei über 100°C und nach dem Abkühlen durch Auftreten eines Vakuums luftdicht abschliessbar sind. Obwohl in solchen Behältern Flüssigkeiten unter sterilen Bedingungen aufbewahrt und transportiert werden können, sind sie für viele Zwecke in der Mikrobiologie und Medizin nicht brauchbar.

Glasgefässe haben den Nachteil, dass sie beim Autoklavieren leicht zerspringen, schwer zu handhaben sind und auch gewichtsmässig bei der erforderlichen mechanischen Stabilität zu schwer werden.

So ist es schwierig, bekannte Behälter mit Sterillösung zu befüllen oder den sterilen Inhalt ohne Gefahr einer Kontamination unter sterilen Bedingungen von einem in einen anderen Behälter, beispielsweise einen Fermenter, zu transferieren. Auch haben die bekannten Behälter den Nachteil, wenn sie Mikroorganismen enthalten, dass diese während der Lagerung des Transportes oder Transfers sedimentieren und so unter sterilen Bedingungen nicht mehr entnehmbar sind.

Eine der Hauptschwierigkeiten liegt bei den bekannten Vorrichtungen in der Abdichtung des Behälters.

So ist ein Sterilbehälter bekannt (DE—A—917 067), dessen Rührorgan aus einer tragenden Stange besteht, die beweglich durch eine Öffnung im Behälterdeckel geführt wird und hermetisch gegen die Öffnung abgedichtet ist. Diese Abdichtung ist in ihrer Konstruktion sehr aufwendig, wird leicht unsteril, ist sehr platzaufwendig, unhandlich und schlecht zu reinigen. An ihrem unteren Ende ist eine gelochte Scheibe angebracht, die durch Vibration der Stange mittels eines Vibrators die Suspension im Behälter in Bewegung hält. Zu- und Ablauföffnungen im Behälter scheinen jedoch den Vorteil der hermetisch abgedichteten Rührstange wieder aufzuheben.

Es ist ein weiterer Sterilbehälter bekannt (DE—A—16 92 034), in dessen Deckel ein Tauchrohr befestigt ist, welches zur Zufuhr von Substratlösung und zur Probeentnahme dient. Das Tauchrohr ist im Deckel verschraubt und in seinem ausserhalb des Deckels befindlichen Teil mit dem unteren Ende einer Substratvorlage verbunden. Es ist in seinem inneren Teil unmittelbar unterhalb des Deckels mit einer verschliessbaren Öffnung versehen und ragt seitlich bis etwa zur Mitte des Behälters.

Nachteile der bekannten Vorrichtungen sind darin zu sehen, dass Bohrungen im Deckel der Gefässe zusätzliche Abdichtungen benötigen und tote Ecken im Bereich der Befestigung auftreten. Zusätzliche Einbauten wie Öffnungen und Verschraubungen erhöhen die Gefahr der Kontamination so, dass Stellen entstehen, die der Reinigung nicht zugänglich sind. Durch wiederholte Sterilisation bei Temperaturen über 121°C brennen vorhandene Eiweissreste so stark ein, dass sie kaum mehr entfernbar sind. Auch die Befestigung des Tauchrohrs in der Nähe der Behälterwand wirkt sich nachteilig auf die Reinhaltung des Gefässes aus. Ausserdem lässt sich dieses schwer demontieren. In der bekannten Vorrichtung ist eine Möglichkeit zur Aufrechterhaltung einer Suspension nicht gegeben.

Ferner ist ein Sterilbehälter aus Glas bekannt (DE—A—2 017 472), an dessen Deckel ein Glasrohr mit einem endseitigen Auflagewulst vorgesehen ist. Ein hufeisenförmiger Magnet mit einer zentralen Bohrung liegt auf diesem Wulst und wird von einem unterhalb des Glasbehälters befindlichen Magnetrührer mit einem entsprechenden, hufeisenförmigen Magnet gedreht. Um den Verschleiss bei grösseren Rührern zu verhindern, wird ein Kugellager verwendet, welches nötigenfalls oberhalb des Flüssigkeitsspiegels angeordnet ist.

Aufgabe der Erfindung ist es, eine Vorrichtung zu schaffen, die die Aufbewahrung, die Inokulation, den Transport und den Transfer von sterilen Lösungen unter Aufrechterhaltung von Suspensionen in besonders einfacher und praktischer Weise gestattet und ein besonders angepasstes Verfahren zum Transfer und/oder zur Probeentnahme von sterilen Lösungen und/oder Suspensionen aus der Vorrichtung zu schaffen.

Diese Aufgabe wird erfindungsgemäss dadurch gelöst, dass die Rührachse als Tauchrohr mit aussenliegendem Ventil ausgebildet ist, dass der Magnetrührkörper mittels einer Steckverbindung und/oder einer Schraubverbindung am feststehenden Tauchrohr lösbar gelagert ist und zwei voneinander getrennte parallel, horizontal und symmetrisch angeordnete Magnestäbe aufweist, und dass jede der nicht zentral gelegenen Bohrungen mit einer gasdichten Gummimembran verschlossen ist zum Einbringen einer Anstechnadel.

Die Bohrung eingebracht wird, so dass eine abdichtende Gummimembrane durchbohrt wird. Mit diesem Verfahren kann eine zusätzliche Zufuhr und/oder Abfuhr von Gasen und Flüssigkeiten durch die Öffnungen im Deckel der erfindungsgemässen Vorrichtung erfolgen.

Das Tauchrohr führt in bevorzugter Weise zentral in den Edelstahlbehälter. Es dient alternativ als Zufuhrleitung für das sterile Substrat, als Entleerungsleitung für steriles Substrat, als Transferleitung in ein anderes Sterilgefäss oder ein Fermentationsgefäss, als Leitung zur direkten Dampfsterilisierung und als Befestigung für einen Magnetrührer. Es kann aber auch als Doppelrohr ausgestaltet sein, in welchem ein Wärmeträger zirkulieren kann.

Das Tauchrohr dient gleichzeitig als Haltevorrichtung für einen Rührkörper. Der Rührkörper ist mittels einer Steckvorrichtung und/oder mittels einer Schraubverbindung im unteren Teil des Tauchrohrs befestigt. Dabei ist es von besonderem Vorteil, wenn der Rührer, bzw. Rührkörper, auf dem feststehenden Tauchrohr einzig um das Tauchrohr rotierbar gelagert ist. Damit ist eine einfache Austauschbarkeit und Reinigung des Rührkörpers gewährleistet.

Diese Mehrzweckverwendung des Tauchrohrs erspart eine Vielzahl an Hilfsgeräten, die bei bekannten Sterilgefässen durch den Deckel geführt werden müssen.

Ein ganz wesentlicher Vorteil liegt in der gasdichten Verbindung des Tauchrohrs mit dem Deckel. Es gelingt damit, den Deckel zusammen mit dem Tauchrohr nach oben zu entfernen, wodurch der Behälter ohne Einbauten leicht zu reinigen ist.

Das Tauchrohr ist fest mit dem Deckel verbunden, so dass keine separaten Dichtungen erforderlich sind. Es ist lediglich eine einzige Dichtung für den Deckel erforderlich, welche jedoch leicht auswechselbar ist. Dichtungen sind aus elastischen Kunststoffen gerfertigt und unterliegen bei häufigen Sterilisierungen einem starken Verschleiss durch Alterung und Bruch. Eine absolute Dichtigkeit von Atmosphäre zum Behälterinhalt ist so nie gewährleistet.

Besonders vorteilhaft hat es sich erwiesen, wenn ein Edelstahltauchrohr direkt mit dem Behälter verschweisst ist. Bei gut ausgeführter Schweissung ist eine absolute Abdichtung gewährleistet.

Der Rührkörper besteht in seiner bevorzugten Ausführung aus zwei Magnetstäben, welche mittels Klemmen in Form von Halbschalen so befestigt sind, dass sie eine symmetrische Einheit bilden, wobei die Magnetstäbe in einer Horizontalen angeordnet sind. Die mittige Öffnung in den Klemmen dient zum Aufschieben des Rührkörpers auf das Tauchrohr. Die parallele Tandemanordnung der Magnetstäbe hat den Vorteil, dass eine turbulente Umwälzung des Behälterinhalts erfolgt, ohne lediglich eine Rotation des Behälterinhalts unter einer Vortexbildung hervorzurufen, wie es bei den bekannten Einstabmagnetrührern der Fall ist.

Der Rührkörper kann auch aus wenigstens einem Rührflügelpaar bestehen, das verschiedene Formen aufweist.

Die Schraubverbindung besteht in ihrer bevorzugten Ausführungsform aus einer Arretiermutter mit einer zentralen Öffnung. Diese Mutter wird zur Befestigung des Rührkörpers am unteren Teil des Tauchrohrs aufgeschraubt und schliesst mit ihrem zylindrischen Teil am Ende des Tauchrohrs ab.

In einer Varianten ist es zweckmässig, die Arretiermutter an ihrer zylindrischen Peripherie mit Öffnungen zu versehen, welche vorzugweise aus Schlitzen bestehen. Das hat den Vorteil, dass im Bedarfsfall der Behälterinhalt belüftet werden kann. Die Öffnungen können aber auch jeder andere geometrische Gestalt aufweisen.

Ebenfalls ist es vorteilhaft, die untere Öffnung des Tauchrohrs so nah wie möglich am Boden des Gefässes enden zu lassen. Zur raschen Entleerung hat sich ein Bodenabstand von 3 bis 5 mm bewährt.

Das Verfahren ist besonders günstig, wenn die Spitze der Anstechnadel lanzettförmig ausgebildet ist und dadurch das mit bekannten Nadeln hervorgerufene Bersten der Gummimembranen, welche beim Anstechen durchbohrt werden müssen, verhindert. Ein weiterer Vorteil ist, dass die lanzettförmigen Spitzen eine senkrechte Perforation gestatten.

Wegen der seitlichen Öffnung im Rohrteil der Anstechnadel können keine ausgestochenen Gummireste in die Öffnung der Nadel eindringen.

Die Erfindung soll anhand von Zeichnungen näher beschrieben werden.

Es zeigen:

Fig. 1 den sterilisierbaren Edelstahlbehälter,

Fig. 2 eine bevorzugte Variante des Rührkörpers,

Fig. 3 eine Arretiermutter für den Rührkörper nach Fig. 4 und

Fig. 4 eine Anstechnadel.

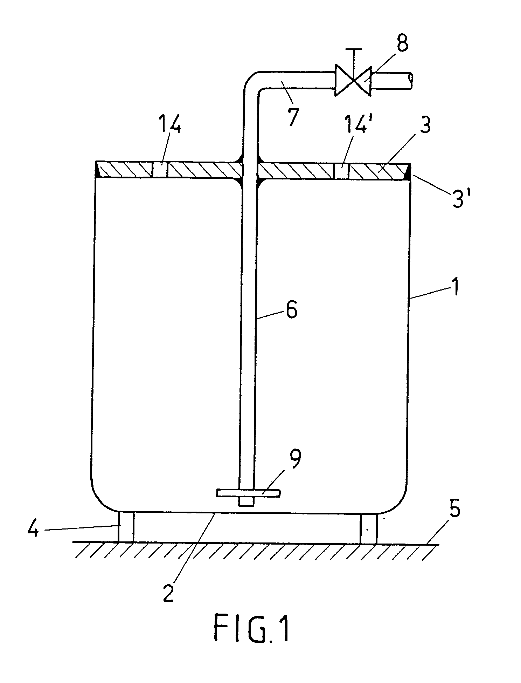

Gemäss Fig. 1 besteht ein Behälter 1 aus Edelstahl, aus einer zylindrischen Seitenwand, einem Boden 2 und einem Deckel 3. Zwischen dem Deckel 3 und dem Behälter 1 ist eine Dichtung 3' vorgesehen. Am Boden 2 des Behälters 1 sind Füsse 4 oder Rollen zum Bewegen des Behälters 1 angebracht. Der Behälter 1 kann als Ganzes auf eine Platte 5 eines an seine Bodenfläche und seine Leistung angepassten Magnetrührers gestellt werden. Im Deckel 3 des Behälters 1 ist ein Tauchrohr 6 befestigt, das in seinem oberen horizontalen Teil 7 durch ein Ventil 8 absperrbar ist. Im unteren Teil des Tauchrohrs 6 ist ein Rührkörper 9 befestigt.

Im Deckel 3 befinden sind mehrere Bohrungen 14, 14', in welche Zu- und/oder Abführleitungen über Anstechnadeln 15 eingebracht werden können.

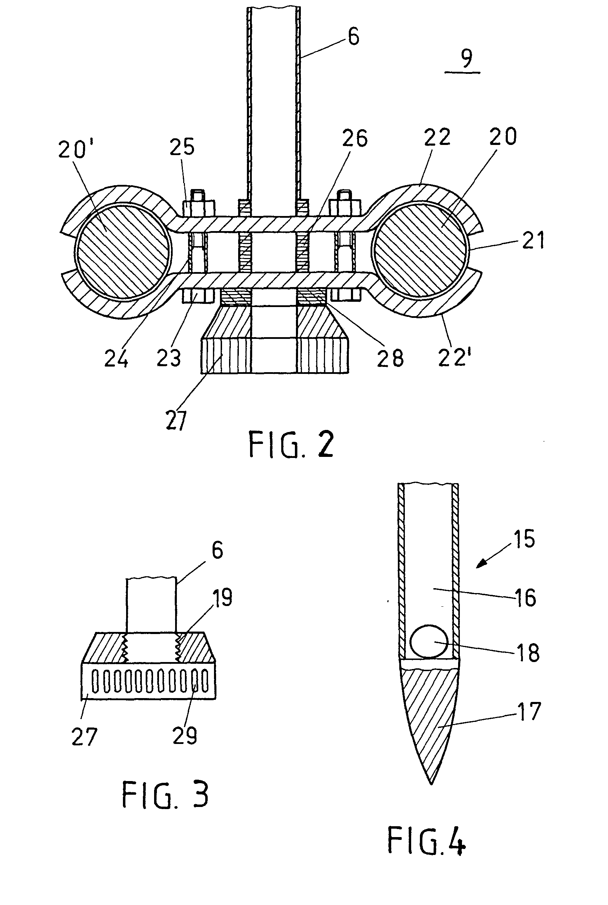

In Fig. 2 ist ein bevorzugter Rührer dargestellt. Der Rührkörper 9 besteht aus zwei zylindrischen Magnetstäben 20 und 20', welche mit einer Schicht 21 aus PTFE versehen sind. Beide Magnetstäbe 20, 20' werden zwischen zwei Klemmen 22, 22', vorzugsweise aus Edelstahl, über Bolzen 23, eine Abstandhülse 24 und eine Mutter 25 gehaltert. Die mittige Bohrung der Klemmen 22, 22' ist mit einer Kunststoffbüchse 26, insbesondere aus PTFE, versehen. Der Rührkörper 9 wird mittels einer mit einem PTFE-Ring 28 versehenen Arretiermutter 27 am Tauchrohr 6 durch Verschraubung befestigt. Der Rührkörper 9 liegt somit auf dem Ring 28 der Arretiermutter 27 durch sein Gewicht auf und ist am Tauchrohr 6 frei drehbar.

In Fig. 3 ist eine Arretiermutter 27 mit Öffnungen 29 in der zylindrischen Peripherie versehen. Die Öffnungen sind in einer bevorzugten Ausführungsform als axial verlaufende Schlitze ausgebildet. Sie können aber auch aus kreisförmigen oder ovalen Öffnungen bestehen. Die Öffnungen dienen zur Begasung eines Mediums.

Gemäss Fig. 4 besteht eine Anstechnadel 15 aus einem Rohr 16, dessen lanzettförmige Spitze 17 kompakt ausgebildet ist. Eine Bohrung 18 mit 2 bis 5 mm, vorzugsweise 4 mm, Durchmesser befindet sich im Rohr 16 im Bereich der Nadelspitze 17. Die Spitze 17 der Nadel 15 ist lanzettförmig abgeflacht.

Im Betrieb wird der Sterilbehälter 1 über das Ventil 8, den oberen waagerechten Teil 7 des Tauchrohrs 6 mit einer Substratlösung befüllt und im Autoklaven sterilisiert. In den Bohrungen 14, 14' können Anstechnadeln 15 angebracht sein, die mit sterilisierbaren Schläuchen versehen sein können und durch Quetschhähne verschlossen werden.

Während der Aufbewahrung von Substrat mit suspendierbaren Teilchen oder Mikroorganismen kann eine Rührung erfolgen, ohne dass eine Dichtung an der Stelle der Durchführung des Tauchrohrs durch den Deckel beansprucht und abgenutzt wird. Auch während des Transports kann mittels des Magnetrührers gerührt und erforderlichenfalls belüftet werden.

Der Transfer einer Sterillösung in einen anderen Behälter oder einen Fermenter kann jederzeit durchgeführt werden, wobei sterilisierte Schläuche mit Anstechnadeln 15 zum Transfer verwendet werden.

Zum Umwälzen des Behälterinhalts hat sich der Tandemmagnetrührer als besonders geeignet erwiesen. Der ganze Rührbehälter kann auf eine Platte eines speziell in seiner Stärke des Magnetfeldes angepassten Magnetrührer gestellt und mit der gewünschten Geschwindigkeit rotiert werden, um den Behälterinhalt in Suspension zu halten. In einem bevorzugten Ausführungsbeispiel, welches zur Verwendung mit Suspensionen mit einer gleichen oder ähnlichen Viskosität wie derjenigen von Wasser vorgesehen ist, kann die Drehzahl des Rührers vom Stillstand bis 800 Umdrehungen pro Minute stufenlos eingestellt werden.

Die erfindungsgemässe Vorrichtung ist palettisierbar. Es können Hilfseinrichtungen wie eine Tarierwaage oder eine Kesselwaschmaschine angeschlossen werden. Der Behälter kann auf Rollen transportiert oder mit einer Aufhängevorrichtung von einer Stelle zu einer anderen verschoben werden.

Zur Verschraubung des Deckels im Behälter hat sich anstelle von Milchverschraubungen der Verwendung eines mit Nocken versehenen Deckels bewährt.

.

## Patentansprüche

1. Vorrichtung zur Aufbewahrung, zum Transport, zum Transfer und zur Probeentnahme von sterilen Lösungen und/oder Suspensionen, insbesondere als Inokuliergefäss, mit einem sterilisierbaren Edelstahlbehälter (1), dessen hermetisch abschliessbarer Deckel (3) mehrere Bohrungen aufweist, wovon ein zentral gelegen ist und eine gasdicht eingelassene hohle Rührachse besitzt, an deren unterem Ende ein Magnetrührkörper (9) drehbeweglich gelagert ist, dadurch gekennzeichnet, dass die Rührachse als Tauchrohr (6) mit aussenliegendem Ventil (8) ausgebildet ist, dass der Magnetrührkörper (9) mittels einer Steckverbindung (12) und/oder einer Schraubverbindung (19) am feststehenden Tauchrohr (6) lösbar gelagert ist und zwei voneinander getrennte parallel, horizontal und symmetrisch angeordnete Magnetstäbe (20, 20') aufweist, und dass jede der nicht zentral gelegenen Bohrungen (14, 14') mit einer gasdichten Gummimembran verschlossen ist zum Einbringen einer Anstechnadel (15).

2. Vorrichtung nach Anspruch 1, dadurch gekennzeichnet, dass das zentrale Tauchrohr (6) mit dem Deckel (3) gasdicht verschweisst ist.

3. Vorrichtung nach Anspruch 1 oder 2, dadurch gekennzeichnet, dass der/die Magnetrührkörper (9 bzw. 20, 20') durch ein Paar Klemmen (22, 22') die eine mittige Bohrung aufweisen, am Tauchrohr befestigt sind.

4. Vorrichtung nach Anspruch 1, dadurch gekennzeichnet, dass die Schraubverbindung (19) aus einer mit einer zentralen Öffnung versehenen Arretiermutter (27) besteht.

5. Vorrichtung nach Anspruch 4, dadurch gekennzeichnet, dass die Arretiermutter (27) an ihrem zylindrischen Teil periphere Öffnungen (29) aufweist.

6. Vorrichtung nach Anspruch 1, dadurch gekennzeichnet, dass die untere Öffnung des Tauchrohrs 3 bis 5 mm vom Boden (2) des Behälters (1) endet.

7. Vorrichtung nach Anspruch 1, dadurch gekennzeichnet, dass die Anstechnadel (15) aus einem Rohrteil (16) und einer massive Spitze (17) besteht.

8. Vorrichtung nach Anspruch 7, dadurch gekennzeichnet, dass die Spitze (17) lanzettförmig ausgebildet ist.

9. Vorrichtung nach einem der vorangehenden Ansprüche, dadurch gekennzeichnet, dass der Rohrteil (16) in unmittelbarer Nähe der Spitze (17) eine Bohrung (18) aufweist.

10. Vorrichtung nach einem der vorangehenden Ansprüche, dadurch gekennzeichnet, dass die Spitze (17) der Nadel (15) abgeflacht ist.

## Revendications

1. Dispositif pour le stockage, le transport, le transfert et la prise d'échantillons de solutions et/ou de suspensions stériles, en particulier comme récipient d'inoculation, avec un récipient stérilisable (1) en acier spécial dont le couvercle (3) pouvant être fermé de manière hermétiquement étanche comporte plusieurs perçages dont l'un est situé au centre et équipé d'un axe d'agitation creux inséré de manière étanche au gaz et à l'extrémité inférieure duquel est monté de manière tournante un corps d'agitation magnétique (9), caractérisé en ce que l'axe d'agitation est conformé en tube plongeur (6) avec une soupape (8) située à l'extérieur; que le corps d'agitation magnétique (9) est monté de manière amovible sur le tube plongeur (6) fixe au moyen d'un connecteur enfichable (12) et/ou d'un raccord à vis (19) et comprend deux barres magnétiques (20, 20') séparées, placées parallèlement, horizontalement et symétriquement l'une par rapport à l'autre; et que chacune des perçages non centraux (14, 14') est obturé au moyen d'une membrane de caoutchouc étanche au gaz pour l'introduction d'une aiguille de percée (15).

2. Dispositif selon la revendication 1, caractérisé en ce que le tube plongeur (6) central est soudé de manière étanche au gaz sur le couvercle (3).

3. Dispositif selon l'une des revendications 1 ou 2, caractérisé en ce que le/les corps d'agitation (9 et respectivement 20, 20') est (sont) fixé(s) sur le tube plongeur par une paire de pinces (22, 22') présentant un alésage central.

4. Dispositif selon la revendication 1, caractérisé en ce que le raccord à vis (19) est constitué par un écrou d'arrêt (27) muni d'une ouverture centrale.

5. Dispositif selon la revendication 4, caractérisé en ce que l'écrou d'arrêt (27) comporte des orifices périphériques (29) sur sa partie cylindrique.

6. Dispositif selon la revendication 1, caractérisé en ce que l'ouverture inférieure du tube plongeur se termine à une distance de 3 à 5 mm du fond (2) du récipient (1).

7. Dispositif selon la revendication 1, caractérisé en ce que l'aiguille de percée (15) se compose d'une partie tubulaire (16) et d'une pointe massive (17).

8. Dispositif selon la revendication 7, caractérisé en ce que la pointe (17) présente une forme lancéolée.

9. Dispositif selon l'une des revendications précédentes, caractérisé en ce que la partie tubulaire (16) présente un alésage (18) à proximité immédiate de la pointe (17).

10. Dispositif selon l'une des revendications précédentes, caractérisé en ce que la pointe (17) de l'aiguille (15) est aplatie.

**Claims**

1. A device for storing, transporting, transfering and taking a sample of sterile solutions and/or suspensions, particularly in the form of an inoculating vessel, having a sterilizable high-grade-steel receptacle (1) whose hermetically sealable cover (3) has a plurality of bores whereof one is situated in the centre and has a hollow stirring-spindle which is inserted in a gas-tight manner and at the lower end of which a magnetic stirring-body (9) is rotatably mounted, characterized in that the stirring spindle is constructed as an immersion tube (6) with an external valve (8), in that the magnetic stirring-body (9) is detachably mounted on the stationary immersion-tube (6) by means of a plug-in connection (12) and/or a screw connection (19) and has two separate magnetic rods (20, 20') which are arranged parallel, horizontal and symmetrical, and in that each of the non-central bores (14, 14') is closed by means of a gas-tight rubber membrane for the introduction of a tapping needle (15).

2. A device in accordance with Claim 1, characterized in that the central immersion-tube (6) is welded in a gas-tight manner to the cover (3).

3. A device in accordance with Claim 1 or 2, characterized in that the magnetic stirring body/bodies (9 and 20, 20' respectively) is/are secured to the immersion tube by means of a pair of clamps (22, 22') which have a central bore.

4. A device in accordance with Claim 1, characterized in that the screw connection (19) is composed of a stop nut (27) with a central opening.

5. A device in accordance with Claim 4, characterized in that the stop nut (27) has peripheral openings (29) on its cylindrical part.

6. A device in accordance with Claim 1, characterized in that the lower opening of the immersion tube ends 3 to 5 mm from the base (2) of the vessel (1).

7. A device in accordance with Claim 1, characterized in that the tapping needle (15) is composed of a pipe part (16) and a solid tip (17).

8. A device in accordance with Claim 7, characterized in that the tip (17) is lancet-shaped in design.

9. A device in accordance with any one of the preceding claims, characterized in that the pipe part (16) has a bore (18) in the immediate vicinity of the tip (17).

10. A device in accordance with any one of the preceding claims, characterized in that the tip (17) of the needle (15) is flattened.

FIG.1

9

FIG. 2

FIG. 3

FIG. 4